# EUROPEAN PATENT SPECIFICATION

(11) **EP 3 399 028 B1**
(45) Date of publication and mention of the grant of the patent: **23.10.2024**
(21) Application number: 16881915.9
(22) Date of filing: 22.07.2016
(51) Int. Cl.: C12N 5/00, C12N 5/079, C12N 5/0797, A61L 27/38, A61L 27/36

(54) **METHOD FOR CULTURING LIMBAL STEM CELLS BY USING AMNIOTIC SLIDE SUPPORT**
VERFAHREN ZUR KULTIVIERUNG VON LIMBUSSTAMMZELLEN UNTER VERWENDUNG EINES AMNIOTISCHEN GLEITTRÄGERS
PROCÉDÉ DE CULTURE DE CELLULES SOUCHES LIMBIQUES À L'AIDE D'UN SUPPORT FORMÉ D'UNE COUPE DE MEMBRANE AMNIOTIQUE

(30) Priority: 31.12.2015 KR 20150190629
(43) Date of publication of application: 07.11.2018
(73) Proprietor: Catholic University Industry Academic Cooperation Foundation, Seoul 06591 (KR)
(72) Inventor: CHUNG, So-Hyang, Seoul 06004 (KR); LEE, Hyun Jung, Seoul 06992 (KR); SEO, Kyung Yul, Seoul 06713 (KR)
(74) Representative: Ter Meer Steinmeister & Partner
(86) International application number: PCT/KR2016/008003
(87) International publication number: WO 2017/115962

(56) References cited:
- WO-A2-2005/079145
- KR-A- 20050 044 392
- KR-A- 20070 001 108
- KR-A- 20080 097 226
- KR-B1- 100 840 979
- NADIA ZAKARIA ET AL: "Standardized Limbal Epithelial Stem Cell Graft Generation and Transplantation", TISSUE ENGINEERING PART C: METHODS, vol. 16, no. 5, 1 October 2010 (2010-10-01), pages 921 - 927, XP055153962, ISSN: 1937-3384, DOI: 10.1089/ten.tec.2009.0634
- SAI KOLLI ET AL: "Successful Clinical Implementation of Corneal Epithelial Stem Cell Therapy for Treatment of Unilateral Limbal Stem Cell Deficiency", STEM CELLS, 1 January 2009 (2009-01-01), pages N/A - N/A, XP055179363, ISSN: 1066-5099, DOI: 10.1002/stem.276
- KRAMEROV, ANDREI A . ET AL.: "Persistence of Reduced Expression of Putative Stem Cell Markers and Slow Wound Healing in Cultured Diabetic Limbal Epithelial Cells", MOLECULAR VISION, vol. 21, 30 December 2015 (2015-12-30), pages 1357 - 1367, XP055513090, Retrieved from the Internet <URL:http://www.molvis.org/molvis/v21/1357>

## Description

### [Technical Field]

The present invention relates to a method for culturing limbal stem cells using an amniotic membrane. More particularly, the present invention relates to a method for effectively increasing/culturing a proportion of stem cells in a limbal tissue-derived epithelial cell sheet by culturing limbal tissues on an amniotic membrane.

### [Background Art]

It has been reported that approximately 4.5 million people worldwide have both eye blindness, and 1 million of them have become blindness due to a corneal disease. The cornea is a structure located at the most anterior part of the eyeball, accounts for approximately 1/6^{th} of the anterior surface area of the eyeball, and has a size of approximately 11 mm and a thickness of generally 0.55 mm. The cornea is thinnest in the center and becomes thicker toward the periphery. In addition, the cornea is transparent tissue, which plays a major role in the refraction and transmission of light and responds sensitively to a foreign substance due to well-developed nerves. The cornea consists of five layers such as the corneal epithelium, Bowman's membrane, corneal stroma, posterior limiting membrane (Descemet's membrane) and corneal endothelium. There are no blood vessels in a normal cornea, and oxygen is provided from air via tears, and nutrients are provided from front aqueous humor and limbus behind the cornea.

The corneal epithelium accounts for approximately 10% of the total thickness of the cornea, extends to the limbal and conjunctival epithelium, and consists of 5 to 7 layers of cells. Bottom cells of the lowermost layer are proliferated and come out of the surface of the cornea, and after 7 to 14 days, the cells are detached. The Bowman's membrane is a membrane consisting of acellular, colorless and transparent fibrils. When once damaged, this membrane is not regenerated. The corneal stroma accounts for approximately 90% of the corneal thickness, mostly consists of collagen fibers, has a uniform size and direction, and is transparent. In addition, the posterior limiting membrane is a thick basement membrane secreted in endothelial cells, and when a person becomes older, the thickness of this membrane is increased. The corneal endothelial cells are flat cuboidal monolayer cells, which are not regenerated after birth.

The survival of the corneal epithelial cells is maintained by stem cells present in the limbus, and limbal stem cells are monofunctional adult stem cells that can differentiate only into the corneal epithelium. In limbal stem cell deficiency, the reduction in migration of basal corneal epithelial cells to the surface and migration of peripheral corneal epithelial cells to the center and the increase in detachment of the corneal epithelial cells, rather than the proliferation thereof, prevent the regeneration of the corneal epithelium. As a result, the conjunctival epithelial cells penetrate the cornea through the limbus, which is called conjunctivalization. Despite a new environment, conjunctival epithelial cells maintain a unique phenotype, and corneal neovascularization occurs, resulting in corneal opacity and blindness.

When the cornea becomes opaque resulting from an inability to maintain transparency due to trauma, severe inflammation or congenital reasons, although all other functions of the eye including the optic nerve are normal, a patient has serious visual impairment. In the case of difficulty in treatment of such opacity with a medication or laser, the cornea needs to be removed and then replaced with a transparent cornea obtained from a donated eye to facilitate the entry of light into the eye, which is accomplished by corneal transplantation. It can be expected that vision recovery caused by such a lesion of the cornea itself is achieved by corneal transplantation, and in the case of conjunctivalization and opacity of the cornea caused by limbal stem cell deficiency, sight restoration can only be expected by successful transplantation of limbal stem cells. Therefore, in this case, since there is no alternative but stem cell transplantation, the corneal opacity and blindness caused by limbal stem cell deficiency are classified as an intractable disease.

A limbal stem cell-deficient disease is a disease that is caused by extensive damage to the limbus due to genetic factors, or acquired factors such as trauma, infection, UV damage, surgical damage, complications caused by wearing of contact lenses and systemic diseases, and thus deficiency of limbal stem cells that can continuously regenerate the corneal epithelium, and the number of patients with limbal stem cell deficiency is continuously increasing because of unreasonable wearing of contact lenses including unapproved cosmetic colored contact lenses, increased prevalence and severity of dry eye, and increased use of toxic eyedrops, in addition to the above-listed causes.

Autologous or allogeneic limbal tissue transplantation has been used as a method for treating limbal stem cell deficiency. For the autologous limbal tissue transplantation, a method for transplanting the same size of limbal tissue extracted from the opposite eye has been used, but a limbal stem cell-deficient disease may occur in the opposite eye. However, in the case of allogeneic limbal tissue transplantation donated from a corpse, patients are suffering from side effects due to the continuous use of a systemic immunosuppressant, and even when a systemic immunosuppressant is used, there is a frequently-occurring problem in that a considerable proportion of stem cells die due to rejection of limbal grafts. Likewise, the limbal stem cell-deficient disease is continuously increasing, but effective and less complicated treatment methods for patients are not properly developed, and therefore there is a need to develop new technologies that increase the success rate of treatment and achieve optimal therapeutic effects.

Specifically, limbal stem cells present in the limbus, which is the contact site of the cornea and conjunctiva, play a pivotal role in regenerating the corneal epithelium while maintaining and repairing the corneal epithelium. However, since the number of the limbal stem cells is less than 1%, and the cells are buried in the basal membrane of the multilayer limbal epithelium, it is very difficult to isolate the cells. To solve such a problem, methods for isolating multifunctional stem cells from the corneal limbus and culturing the cells have been reported (Korean Patent No. 10-0931887), but there is no effective method that can be used in clinical and practical uses.

WO 2005/079145 A2 relates to a tissue system with self-regenerating limbal stem cells, wherein the limbal stem cells are primarily undifferentiated stem cells (USCs).

N. Zakaria et al., "Standardized Limbal Epithelial Stem Cell Graft Generation and Transplantation", TISSUE ENGINEERING PART C: METHODS, (20101001), vol. 16, no. 5, pages 921 - 927 describes a method for the standardized generation of a limbal epithelial stem cell graft.

### [Disclosure]

### [Technical Problem]

For this reason, the inventors had continuously conducted a study on an effective method for ex vivo expansion of limbal stem cells, and thus completed the present invention.

Therefore, the present invention is directed to providing a method for effectively increasing the proportion of limbal stem cells in a limbal tissue-derived epithelial cell sheet, so that the success rate of transplantation is maximized by transplanting the limbal tissue-derived epithelial cell sheet into a patient with reduced vision and blindness due to limbal stem cell deficiency.

However, technical problems to be solved in the present invention are not limited to the above-described problems, and other problems which are not described herein will be fully understood by those of ordinary skill in the art from the following descriptions.

### [Technical Solution]

To achieve the object of the present invention, the present invention provides a method for culturing limbal stem cells from limbal tissue, comprising: covering a slide glass with an epithelial cell-removed amniotic membrane to obtain a slide glass scaffold for fixation; and culturing limbal tissue on the fixed amniotic membrane, wherein the limbal tissue is cultured in DMEM/F12(1:1) supplemented with human serum added at 4 to 6%(v/v) of the entire medium, an epithelial cell growth factor (EGF), dimethyl sulfoxide (DMSO), insulin transferrin selenium (ITS) and O-phosphoethanolamine.

In an embodiment of the present invention, each of the width and the length of the amniotic membrane is 28 to 35 mm.

In another embodiment of the present invention, epithelial cells of the amniotic membrane were removed using 4 to 6 M urea.

In still another embodiment of the present invention, each of the width and the length of the slide glass is 18 to 28 mm.

In yet another embodiment of the present invention, the limbal tissue is cultured for 10 to 14 days.

In yet another embodiment of the present invention, the limbal tissue-derived epithelial cell sheet is grown to 85 to 95% of the area of the scaffold, and then is classified as a transplant for a patient.

In yet another embodiment of the present invention, the human serum is human AB serum.

### [Advantageous Effects]

When an amniotic membrane slide scaffold and a specific culture condition according to the present invention are used, the proportion of limbal stem cells in a limbal tissue-derived epithelial cell sheet can be stable and rapidly increased. Therefore, the success rate of transplantation can be increased when the limbal tissue-derived epithelial cell sheet is transplanted into a limbal stem cell-deficient patient.

In addition, in the present invention, the proportion of limbal stem cells effective for compatibility in transplantation can be confirmed.

### [Description of Drawings]

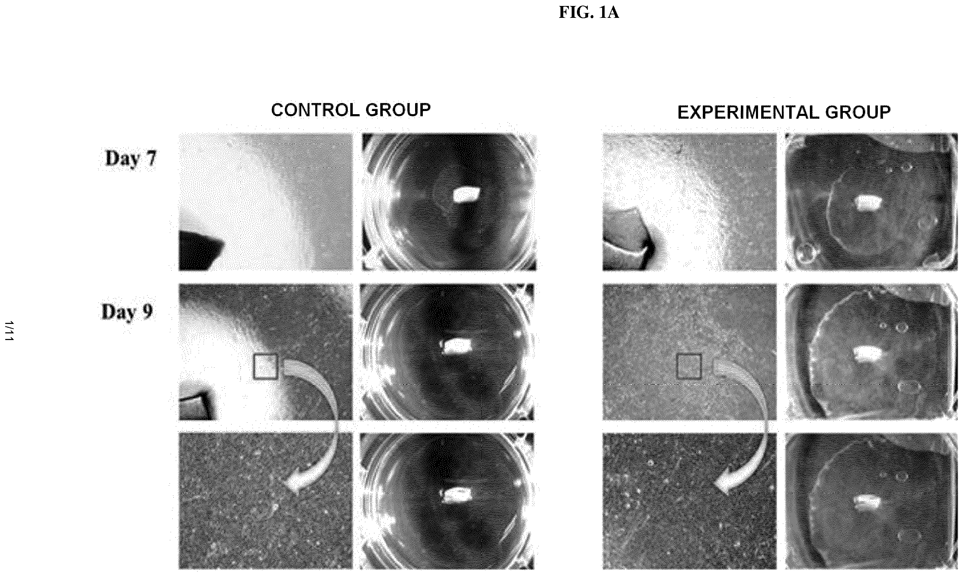

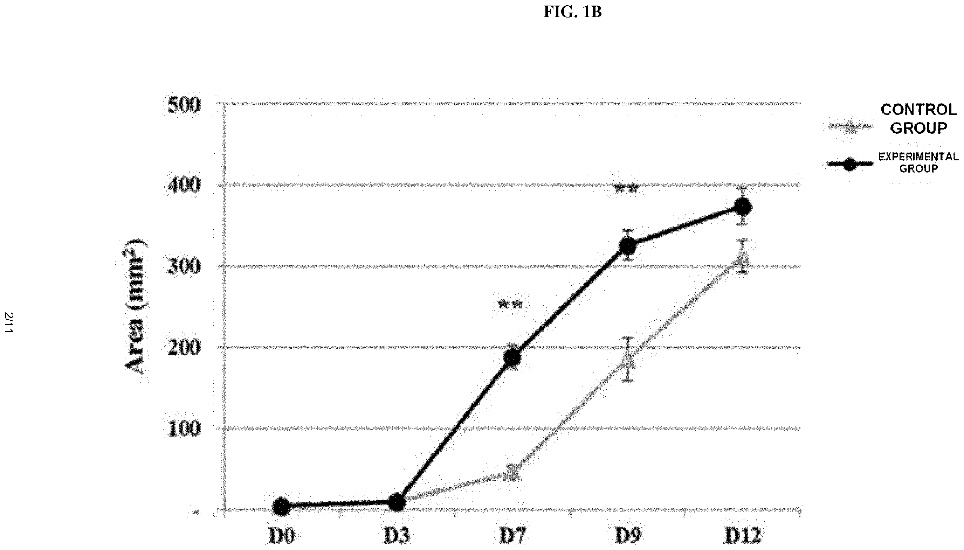

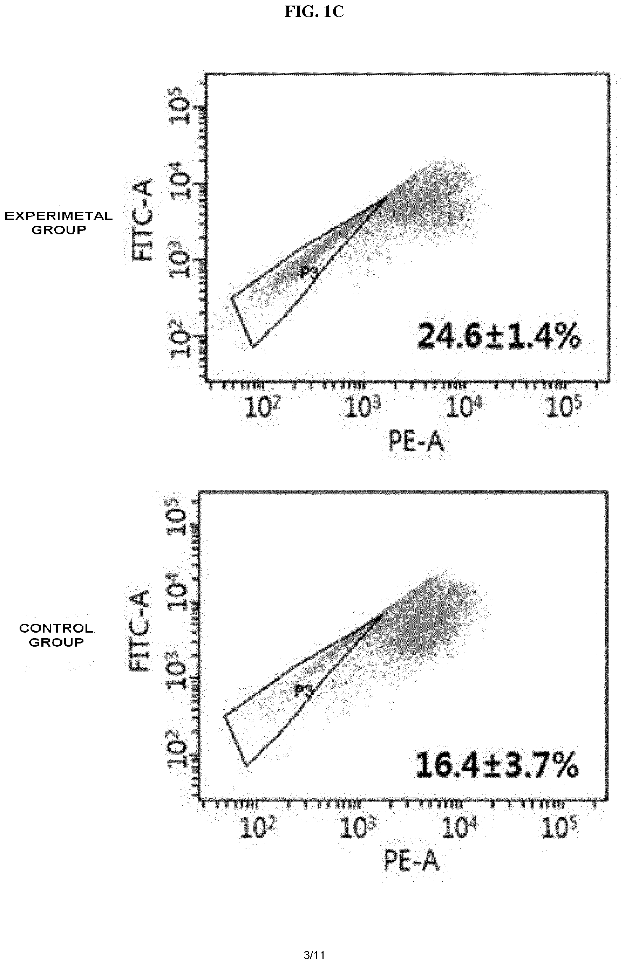

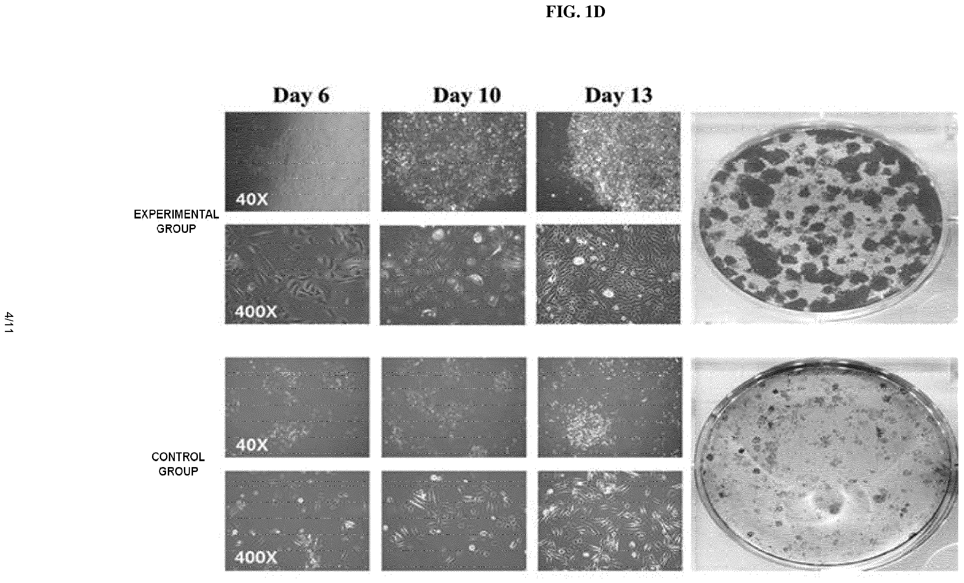

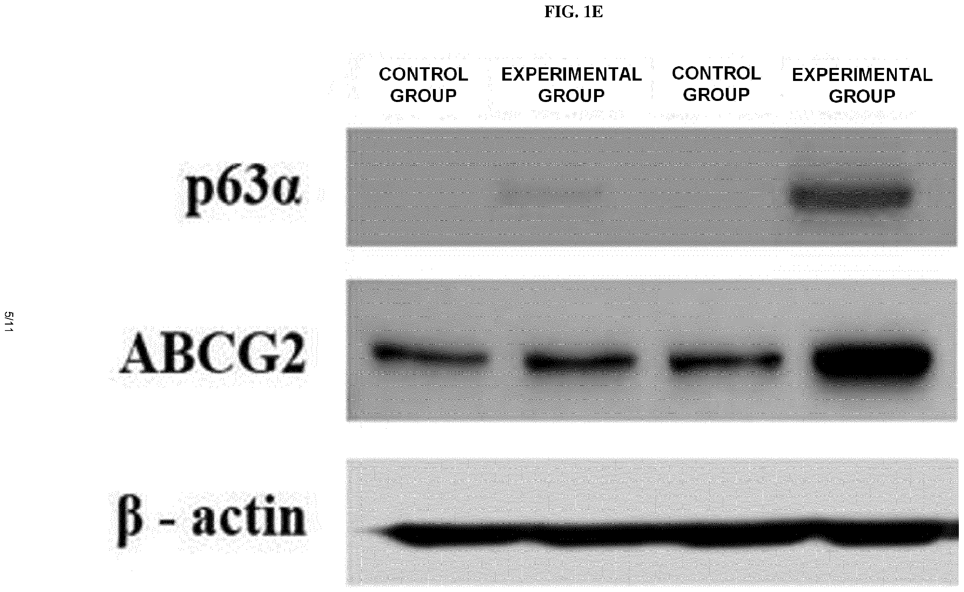

FIGS. 1A to 1E show cell morphology (FIG. 1A), a cell proliferation area (FIG. 1B), a JC-1 low population, which is the proportion of limbal stem cells, (FIG. 1C) and colony forming efficiency (CFE) (FIG. 1D), and a western blotting result for limbal stem cell markers (p63α(+) and ABCG2(+)) (FIG. 1E) in an experimental group in which limbal tissues are cultured on an amniotic membrane slide scaffold prepared according to an exemplary embodiment of the present invention using a supplemented human epithelium medium (SHEM) prepared by adding EGF, DMSO, ITS, O-phosphoethanolamine, cholera toxin (CT) and fetal bovine serum (FBS) to DMEM:F-12(1:1), and a control group in which limbal tissues are cultured on a transwell.

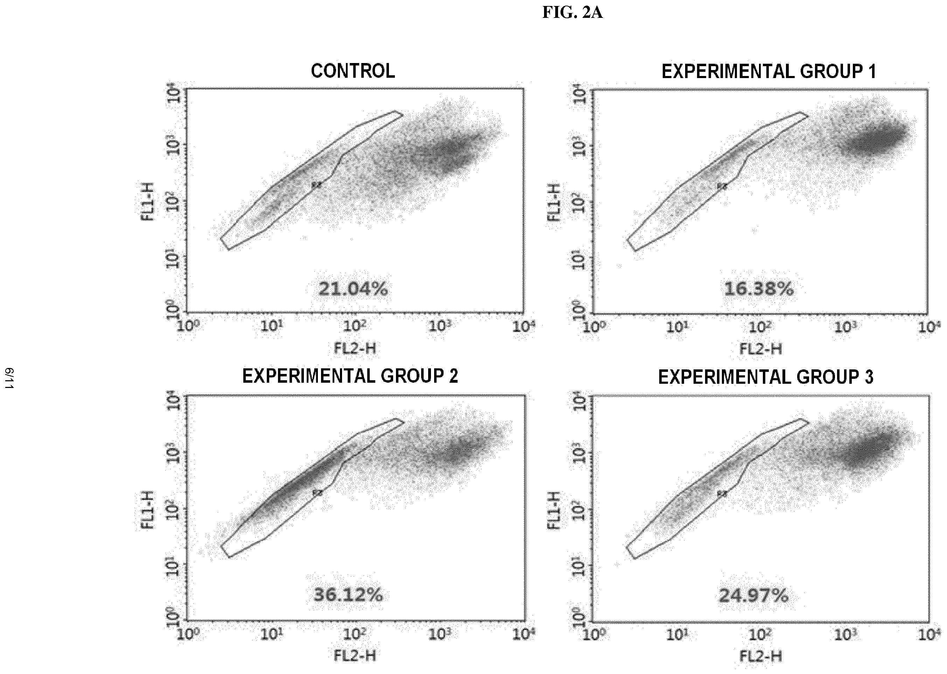

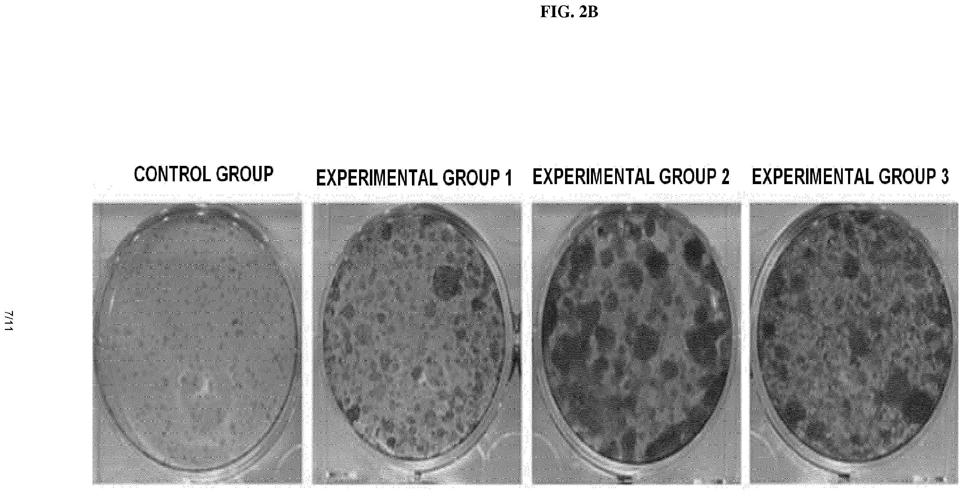

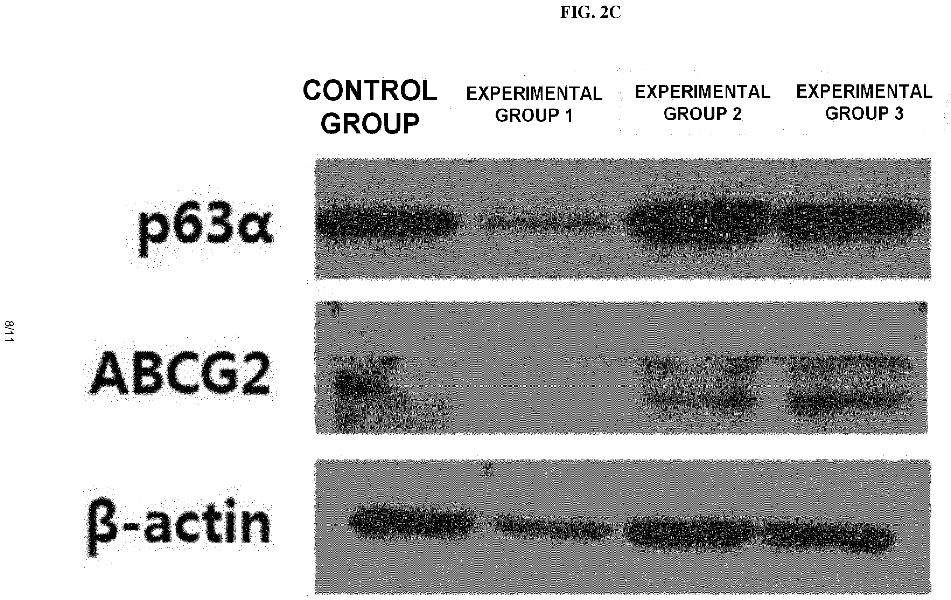

FIGS. 2A to 2C shows the comparison of a JC-1 low population, which is the proportion of limbal stem cells, (FIG. 2A), CFE (FIG. 2B), and a western blotting result for limbal stem cell markers (p63α(+) and ABCG2(+)) (FIG. 2C) in Experimental Groups 1 to 3 in which limbal tissues are cultured using SHEM containing human AB sera (5% and 10%) except CT and FBS on an amniotic membrane slide scaffold prepared according to an exemplary embodiment of the present invention (Control Group : SHEM used in FIG. 1 of the present invention).

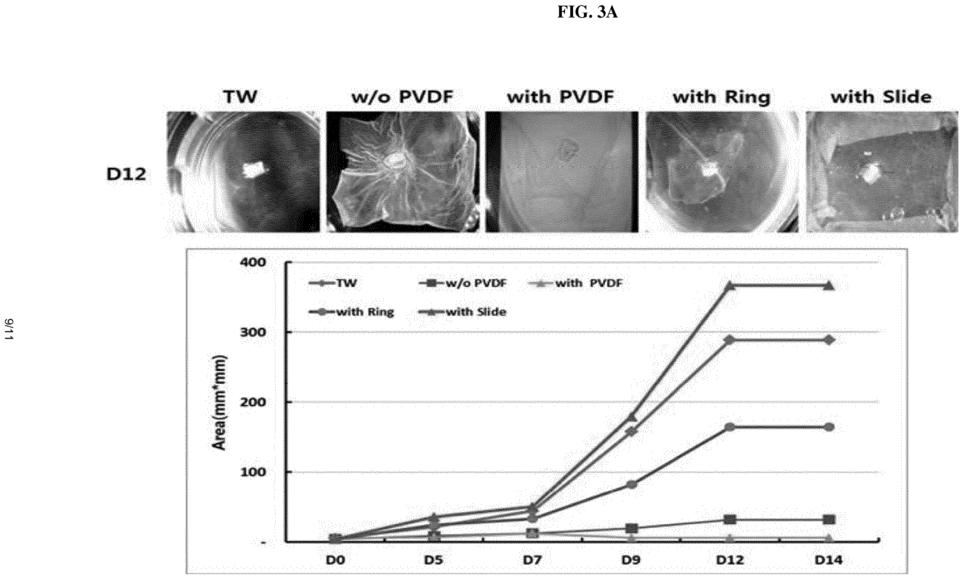

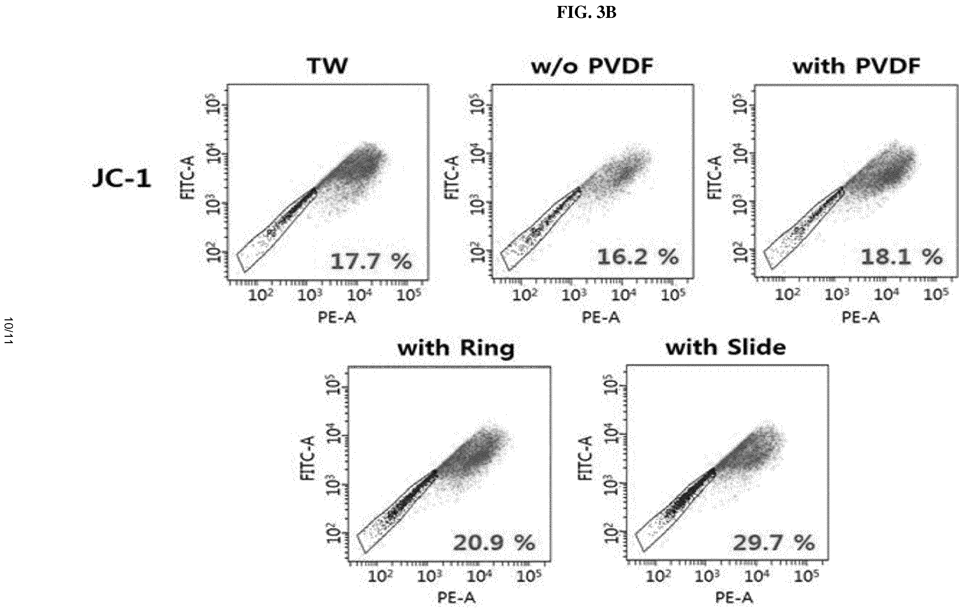

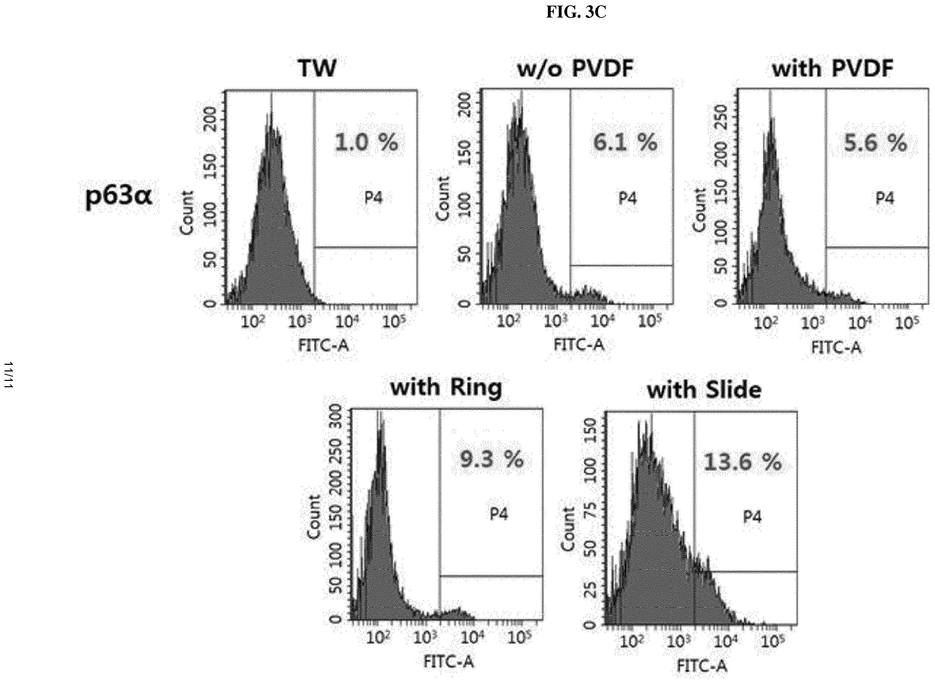

FIGS. 3A to 3C show a cell proliferation area (FIG. 3A) and a JC-1 low population (FIG. 3B), and a flow cytometry result for a limbal stem cell marker (p63α(+)) (FIG. 3C) in an experimental group (with slide) in which limbal tissues are cultured on an amniotic membrane slide scaffold prepared according to an exemplary embodiment of the present invention using SHEM (Experimental Group 2) containing 5% human AB serum except CT and FBS, which is used in FIG. 2 of the present invention; and control groups in which limbal tissues are cultured on a transwell (TW), an amniotic membrane unattached to a PVDF membrane (w/o PVDF), an amniotic membrane attached to a PVDF membrane (with PVDF) and an amniotic membrane fixed with a ring (with ring).

### [Modes of the Invention]

Hereinafter, the present invention will be described in detail.

The present invention relates to a method for effectively proliferating and culturing the proportion of limbal stem cells in a limbal tissue-derived epithelial cell sheet by culturing limbal tissues on a slide scaffold fixed to an amniotic membrane under a specific culture condition.

The limbus is a circular region having a width of approximately 1 mm between the cornea and the sclera, includes many blood vessels in a matrix underlying multilayer epithelial cells, and is involved in metabolism and integration of the cornea. Particularly, since limbal stem cells (ABCG2 positive and p63α positive) in the limbal epithelium are the source of the corneal epithelial cells, when limbal tissue is transplanted into a patient with a limbal stem cell deficient disease, corneal epithelial cells are differentiated from limbal stem cells distributed in a limbal epithelium basal layer and migrate to the corneal center, thereby forming a multilayer corneal epithelium.

Therefore, the inventors had conducted a study on a method for increasing a transplantation success rate by dividing circular limbal tissue of a donor, which remains after corneal transplantation, into 12 pieces, and culturing limbal stem cells in a limbal tissue-derived epithelial cell sheet to contain the limbal stem cells at a predetermined ratio or more from limbal tissue using a slide with an amniotic membrane scaffold, resulting in completion of the present invention.

First, limbal tissue is preferably cultured on an amniotic membrane scaffold, following the division of the circular limbus of a donor into 12 pieces by surgery to so as to have a size of 2×3 mm (major axis × length). The amniotic membrane scaffold is prepared by covering a fixable scaffold with an amniotic membrane. Any plate-type scaffold that can fix the amniotic membrane may be used, and particularly, a slide glass is preferably used.

Here, the amniotic membrane may have a size of 28 to 35 mm (width × length), and particularly, an amniotic membrane in which each of the width and length is 20 mm or more is most preferable because, considering the size of a human cornea (approximately 12×12 mm), limbal tissue with a most suitable size for transplantation can be cultured thereon.

The slide glass having a size of 20 mm × 26 mm (width × length) is most suitable for being stably fixed to the center of a 30 mm culture dish after being covered with the preferable size of amniotic membrane.

The amniotic membrane slide scaffold is located in the center of the 30 mm culture dish, limbal tissue is settled in the middle of the amniotic membrane slide scaffold, and incubated in a culture dish containing a culture medium in a CO₂ incubator at 37°C. Here, the limbal tissue is divided into 12 pieces each having a major axis length of 2 to 3 mm, which is preferable to increase the proportion of the limbal stem cells in the limbal tissue-derived epithelial cell sheet when being cultured in the middle of the amniotic membrane scaffold.

A culture medium is DMEM/F12(1:1) supplemented with human serum added at 4 to 6% (v/v) of the entire medium, an epithelial cell growth factor (EGF), dimethyl sulfoxide (DMSO), insulin transferrin selenium (ITS) and O-phosphoethanolamine.Unless particularly described otherwise, the present invention uses DMEM/F-12 (1:1) supplemented with EGF, DMSO, ITS, O-phosphoethanolamine and human AB serum (5%).

A culture medium of the limbal tissue is preferably changed every 2 to 3 days, and the limbal tissue is grown until a limbal tissue-derived epithelial cell sheet formed through cell division accounts for 85 to 95% of the amniotic membrane scaffold to reach 250 mm² to 300 mm², which is suitable in consideration of the size of the human cornea. A period in which the limbal tissue-derived epithelial cell sheet accounts for 85 to 95% of the amniotic membrane scaffold is approximately 10 to 14 days, and generally a 12-day culture period is needed.

Since the proportion of limbal stem cells present in a limbal tissue-derived epithelial cell sheet is very important for increasing a success rate after transplantation, as a result of confirming the optimal proportion of stem cells in the present invention, it was revealed that at least 30% (38.7± 3.52) stem cells are present in an epithelial cell sheet to be transplanted. Here, the proportion of limbal stem cells may be confirmed as a 5,5',6,6'-tetrachloro-1,1',3,3'-tetraethylbenzimidazol-carbocyanine iodide (JC-1) low population, sternness of the limbal stem cells may be identified by colony forming efficiency (CFE) and western blotting for limbal stem cells-positive markers (p63α and ABCG2), but the present invention is not limited thereto.

In addition, when a specific composition of culture medium and the amniotic membrane slide scaffold according to the present invention are used (with slide), compared with other conditions (TW, without PVDF, with PVDF, and with ring), it can be confirmed that a faster cell proliferation rate and a higher proportion of limbal stem cells are exhibited (refer to FIGS. 3A to 3C).

Hereinafter, to help in understanding the present invention, exemplary examples will be suggested. However, the following examples are merely provided to more easily understand the present invention, and not to limit the present invention.

### [Examples]

### Example 1: Preparation of amniotic membrane slide scaffold

Amniotic membrane tissue (30 mm × 30 mm) that had been provided from a human tissue bank and then cryopreserved at -70°C was left at room temperature for 30 minutes, and then epithelial cells of the amniotic membrane were removed by treating 5M urea for 5 minutes. A slide glass having a size of 20 mm × 26 mm is covered with the epithelial cell-removed amniotic membrane, and then the slide glass was put into a 35 mm culture dish and tightly fixed.

### Example 2: Culture of limbal stem cells using amniotic membrane slide scaffold

To extract limbal tissue from a corpse after corneal transplantation surgery had been finished, the limbal tissue was divided into 12 pieces each having a size of approximately 2 mm × 3 mm using a surgical tool, and the peripheral cornea and conjunctiva were dissected and removed. Afterward, the extracted limbus was put on the amniotic membrane slide scaffold prepared in Example 1 and cultured for approximately 12 days at 37°C in a CO₂ incubator. In the incubation, a culture medium was changed every 2 to 3 days, and when a limbal tissue-derived epithelial cell sheet accounted for 85 to 90% or more of the slide area, it was transplanted into a patient.

Here, the culture medium was a supplemented human epithelium medium (SHEM) which is conventionally and generally used to culture limbal tissue, and its composition includes EGF, DMSO, ITS, O-phosphoethanolamine, CT and FBS in DMEM:F-12(1:1).

### Example 3: Comparative analysis of proportions of limbal stem cells on amniotic membrane slide scaffold and transwell

To comparatively measure the proportions of limbal stem cells in limbal tissue-derived epithelial cell sheets, cells grown to 90% or more on an amniotic membrane slide scaffold and a transwell (control group) were treated with 2 mg/ml of a dispase II solution at 4°C overnight. The limbal tissue-derived epithelial cell sheet was isolated from the amniotic membrane using fine forceps, treated with 1 ml of trypLE for 5 minutes, and centrifuged at 1000 rpm for 5 minutes, thereby obtaining a cell precipitate. The cell precipitate was suspended in SHEM, and then cells were counted to measure a JC-1 low population and CFE, and seeded in a 6-well plate. To measure the JC-1 low population, the cells were seeded at 1.0 × 10⁵ cells/ml, cultured in SHEM for 24 hours and treated with a JC-1 dye for 1 hour, followed by measuring a JC-1 low cell population, which are side population cells, using FACS. Meanwhile, to confirm colony formation, the cells were cultured in a CNT50 medium (Cellntec) for 14 days, and the number of colonies was counted, thereby confirming CFE.

Consequently, as confirmed in FIGS. 1A to 1E, when the amniotic membrane slide scaffold prepared according to the present invention was used, a proliferation rate of the cells was significantly increased, compared to the transwell, which is the control group, and the JC-1 low population, which represents the proportion of stem cells included in the limbal tissue, CFE and expression of stem cell markers (p63α and ABCG2) were also significantly increased.

### Example 4: Comparison of efficiencies according to medium composition

Conventionally, CT and FBS were included in SHEM which has been generally used to culture limbal tissue. However, the cultured limbal tissue is for use in human transplantation, and therefore, in order to exclude an animal component (FBS) and a toxicity component (CT) in the culture, a culture experiment was carried out with the medium compositions shown in Table 1.

**[Table 1]**

| **Control** | **Experimental Group 1** | **Experimental Group 2** | **Experimental Group 3 (Reference Example)** |
|---|---|---|---|
| SHEM | SHEM without CT | SHEM with human AB serum (5%) without CT | SHEM with human AB serum (10%) without CT |

Consequently, as confirmed in FIGS. 2A to 2C, in Experimental Group 1 from which CT was removed, the proportion of limbal stem cells was slightly reduced, and in Experimental Group 2 using 5% human albumin serum (AB serum) instead of 5% animal serum (FBS), the proportion of limbal stem cells and expression of stem cell markers were significantly increased. Meanwhile, in Experimental Group 3 in which the ratio of a human AB serum was increased to 10%, compared to the case of adding 5% human AB serum, the proportion of limbal stem cells in a limbal tissue-derived epithelial cell sheet was decreased to the level of the control group. Therefore, it can be seen that the medium condition in Experimental Group 2 was most preferable.

### Example 5: Comparative analysis of proportions of limbal stem cells in various scaffolds

To compare the proportions of limbal stem cells in various scaffolds, on day 5, 7, 9, 12 and 14 of culturing, cell proliferation areas and the proportions of limbal stem cells were measured in the cases of the transwell (TW), the amniotic membrane not attached to a PVDF membrane (w/o PVDF), the amniotic membrane attached to a PVDF membrane (with PVDF), the amniotic membrane fixed with a ring (with ring) as the control groups, and the amniotic membrane attached to the slide glass of the present invention (with slide).

This experiment was carried out in the same manner as used in Example 3, except that the medium condition for Experimental Group 2 described in Example 4, instead of SHEM, was used.

Consequently, as confirmed in FIGS. 3A to 3C, when the amniotic membrane slide scaffold prepared according to the present invention was used, compared with the control groups, a cell proliferation rate was considerably increased (FIG. 3A), and as a result of observing a JC-1 low population and flow cytometry for a limbal stem cell marker (p63α(+))(FIGS. 3B and 3C), it can be seen that the amniotic membrane slide scaffold was most preferable.

### [Industrial Applicability]

A method for culturing limbal stem cells using an amniotic membrane slide scaffold according to the present invention may allow the proportion of the limbal stem cells in a limbal tissue-derived epithelial cell sheet to be stably and rapidly increased, and thus this method is expected to be useful in treatment of a patient with limbal stem cell deficiency through limbal stem cell transplantation.

## Claims

1. A method for culturing limbal stem cells from limbal tissue, comprising:
covering a slide glass with an epithelial cell-removed amniotic membrane to obtain a slide glass scaffold for fixation; and
culturing limbal tissue on the fixed amniotic membrane,
wherein the limbal tissue is cultured in DMEM/F12(1:1) supplemented with human serum added at 4 to 6%(v/v) of the entire medium, an epithelial cell growth factor (EGF), dimethyl sulfoxide (DMSO), insulin transferrin selenium (ITS) and O-phosphoethanolamine.

2. The method according to claim 1, wherein each of the width and length of the amniotic membrane is 28 to 35 mm.

3. The method according to claim 1, wherein epithelial cells of the amniotic membrane are removed using 4 to 6 M urea.

4. The method according to claim 1, wherein each of the width and length of the slide glass is 18 to 28 mm.

5. The method according to claim 1, wherein the limbal tissue is cultured for 10 to 14 days.

6. The method according to claim 5, wherein, when the limbal tissue is grown to 85 to 95% of the area of the scaffold, the limbal tissue is classified as a transplant for a patient.

7. The method according to claim 1, wherein the human serum is human AB serum.

## Patentansprüche

1. Verfahren zum Kultivieren von limbalen Stammzellen von Limbusgewebe, das umfasst:
Bedecken eines Objektglases mit einer von Epithelzellen befreiten amniotischen Membran, um ein Objektglasgerüst zur Fixierung zu erhalten; und
Kultivieren von Limbusgewebe auf der fixierten amniotischen Membran,
wobei das Limbusgewebe in DMEM/F12(1:1), das mit Humanserum, das mit 4 bis 6% (v/v) des gesamten Mediums hinzugefügt wird, einem Wachstumsfaktor für Epithelzellen (EGF), Dimethylsulfoxid (DMSO), Insulintransferrinselen (IST) und O-Phosphoethanolamin ergänzt ist.

2. Verfahren nach Anspruch 1, wobei jede der Breite und der Länge der amniotischen Membran 28 bis 35 mm ist.

3. Verfahren nach Anspruch 1, wobei Epithelzellen der amniotischen Membran unter Verwendung von 4 bis 6 M Urea entfernt werden.

4. Verfahren nach Anspruch 1, wobei sowohl die Breite als auch die Länge des Objektglases 18 bis 28 mm beträgt.

5. Verfahren nach Anspruch 1, wobei das Limbusgewebe für 10 bis 14 Tage kultiviert wird.

6. Verfahren nach Anspruch 5, wobei das Limbusgewebe dann, wenn es bis zu 85 bis 95 % der Fläche des Gerüsts gewachsen ist, als ein Transplantat für einen Patienten klassifiziert wird.

7. Verfahren nach Anspruch 1, wobei das Humanserum menschliches AB-Serum ist.

## Revendications

1. Procédé de culture de cellules souches limbiques provenant de tissu limbique, comportant les étapes consistant à :
recouvrir une lame de verre d'une membrane amniotique dont les cellules épithéliales ont été retirées pour obtenir un support de lame de verre en vue d'une fixation ; et
mettre en culture du tissu limbique sur la membrane amniotique fixée,
dans lequel le tissu limbique est mis en culture dans du DMEM/F12 (1:1) complété par du sérum humain ajouté à 4 à 6 % (v/v) du milieu complet, un facteur de croissance des cellules épithéliales (EGF), du sulfoxyde de diméthyle (DMSO), de l'insuline-transferrine-sélénium (ITS) et de l'O-phosphoéthanolamine.

2. Procédé selon la revendication 1, dans lequel chaque dimension parmi la largeur et la longueur de la membrane amniotique est de 28 à 35 mm.

3. Procédé selon la revendication 1, dans lequel des cellules épithéliales de la membrane amniotique sont retirées en utilisant de l'urée 4 à 6 M.

4. Procédé selon la revendication 1, dans lequel chaque dimension parmi la largeur et la longueur de la lame de verre est de 18 à 28 mm.

5. Procédé selon la revendication 4, dans lequel le tissu limbique est mis en culture pendant 10 à 14 jours.

6. Procédé selon la revendication 5, dans lequel, lorsque le tissu limbique a proliféré sur 85 à 95 % de la surface du support, le tissu limbique est classé comme un transplant destiné à un patient.

7. Procédé selon la revendication 1, dans lequel le sérum humain est le sérum AB humain.
